(19) Europäisches Patentamt / European Patent Office / Office européen des brevets

(11) **EP 3 022 547 B1**

(12) **EUROPEAN PATENT SPECIFICATION**

(45) Date of publication and mention
of the grant of the patent:
**12.12.2018 Bulletin 2018/50**

(21) Application number: **14738835.9**

(22) Date of filing: **14.07.2014**

(51) Int Cl.:
*G01N 21/64* *(2006.01)*          *G01N 33/543* *(2006.01)*
*G01N 33/551* *(2006.01)*          *G01N 21/47* *(2006.01)*

(86) International application number:
**PCT/EP2014/065013**

(87) International publication number:
**WO 2015/007674 (22.01.2015 Gazette 2015/03)**

(54) **DEVICE FOR USE IN THE DETECTION OF BINDING AFFINITIES**

VORRICHTUNG ZUR VERWENDUNG BEI DER ERKENNUNG VON BINDUNGSAFFINITÄTEN

DISPOSITIF À UTILISER POUR LA DÉTECTION DES AFFINITÉS DE LIAISON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.07.2013 EP 13176536**

(43) Date of publication of application:
**25.05.2016 Bulletin 2016/21**

(73) Proprietor: **F. Hoffmann-La Roche AG
4070 Basel (CH)**

(72) Inventor: **FATTINGER, Christof
CH-4223 Blauen (CH)**

(74) Representative: **Bohest AG
Holbeinstrasse 36-38
4051 Basel (CH)**

(56) References cited:
EP-A1- 2 618 130          US-A- 4 647 544
US-A- 4 876 208          US-A- 4 931 384
US-A- 5 124 172          US-A1- 2002 025 534
US-A1- 2006 160 245      US-B1- 6 483 096

**Description**

The present invention relates to a device for use in the detection of binding affinities between a binding site and a target molecule and to a method for detecting binding affinities.

Optical biosensors are devices which enable the detection of binding affinities. The binding affinity refers to the strength of the molecular interaction (e.g. a high binding affinity results from a greater intermolecular force between the binding site and the target molecule). A typical field of application for such optical biosensors is the detection of binding affinities between receptor molecules (as binding sites) and a predetermined target molecule without limitation to any specific chemical, biological or pharmaceutical substance of interest. This detection can be carried out in a sandwich-assay in which the target molecule has bound to the binding site and to a labelled complementary binder molecule. The light emanating from the label is measured to detect the binding affinities.

Alternatively, the capability of the target molecule to bind to the receptor molecule can be detected in a label-free manner. For example, in Surface Plasmon Resonance Spectroscopy (SPR) an optical biosensor enables the label-free detection by spectroscopically measuring a resonance shift in an absorption spectrum. The spectroscopic signal characteristically changes if a target molecule has bound to the attached receptor molecules and this change is representative of the binding affinity.

A state of the art SPR-device comprises a transparent substrate comprising at one side thereof a metal layer (e.g. a thin gold layer). A prism is arranged on the side of the substrate opposite to the side where the metal layer is arranged. Receptor molecules acting as binding sites are immobilized on the metal layer. Target molecules are then applied to the receptor molecules for the detection of the binding affinity between the receptor molecules and the target molecules.

During use of such device, an incident beam of light is directed via the prism through the transparent substrate onto the metal layer. The incident beam of light electromagnetically couples with a surface plasmon. The surface plasmon is a coherent electron oscillation which occurs at the interface of the metal and the medium above the outer surface of the metal, and which propagates along the metal layer. The plasmon changes if attached receptor molecules have bound to applied target molecules. In terms of physics, it is the resonance frequency of the propagating surface plasmon that changes characteristically in relation to binding events occurring between the receptor molecules and the target molecule. The reflected portion of the incident light is spectroscopically analysed by measuring changes in an angular absorption spectrum which provides a signal representative of occurring binding events between the receptor molecules and the target molecules.

A disadvantage associated with the above described SPR-device is the need for a metal layer to which the target molecule may bind unspecifically. Another disadvantage is that the sensitivity is limited by changes of the refractive index which accumulate over the propagation path of the plasmon.

EP 2 618 130 A1 discloses a device and method for detection of binding affinities of target sample molecules to specific binding sites comprising corresponding capture molecules arranged on a substrate. A grating is used to couple coherent light into a planar waveguide of the substrate to allow propagation to a measurement zone in which the capture molecules are arranged in lines e.g. fifty lines of binding sites.

US 2002/025534 A1 discloses a diagnostic sensing device for detecting analytes in a medium using diffraction-based techniques. Sample analytes are printed onto a substrate, in a defined pattern, such that the substrate leads to diffraction of light reflected off of or transmitted through it which depends upon whether an analyte interacting with the substrate binds to it. The diffraction may increase or decrease on binding of the analyte.

It is an object of the present invention to provide a device for use in the detection of binding affinities between binding sites and a target molecule which overcomes or at least greatly reduces the disadvantages associated with prior art devices.

In accordance with the invention, this object is achieved by a device for use in the detection of binding affinities. The device comprises a substrate devoid of a waveguide, the substrate comprising a planar surface having arranged thereon a plurality of binding sites capable of binding with a target molecule. The binding sites are arranged on the planar surface along a plurality of adjacently arranged curved lines which are spaced from one another by a distance to in operation cause a beam of coherent light of a predetermined wavelength generated at a predetermined beam generation location relative to the plurality of adjacently arranged curved lines and incident on the binding sites to be diffracted at the binding sites with the bound target molecule in a manner such that diffracted portions of the incident beam of coherent light interfere at a predetermined detection location relative to the plurality of adjacently arranged curved lines with a difference in optical path length which is a multiple integer of the predetermined wavelength of the coherent light to provide at the detection location a signal representative of the binding affinity of the binding sites and the target molecule. The device further comprises a beam stop which is arranged to prevent propagation of non-diffracted portions of the incident beam of coherent light to the predetermined detection location.

For the detection of binding affinities, the binding sites are arranged along the plurality of curved lines and the target molecule is applied to the binding sites. In general, "binding sites" are locations on the planar surface to which a target molecule may bind (or binds in case of binding affinity). The detection of binding affinities according to the invention is neither limited to any specific type of target molecules nor to any type of binding sites, but rather the binding characteristics of molecules, proteins, DNA, etc. as target molecules can be analysed with respect to any suitable type of binding sites on the planar surface. The term diffracted "portion" of the incident beam refers to the fact that it is not the entire incident beam of coherent light which is diffracted so that a portion of the incident beam (in fact the main portion of the incident beam) continues to propagate in the direction of the incident beam. The incident beam of coherent light of a predetermined wavelength is generated at the predetermined beam generation location and may be generated by a laser light source. It propagates to impinge on the curved lines so that a portion thereof is diffracted and propagates towards the predetermined detection location. The diffracted portions of the incident beam of coherent light interfere at the predetermined detection location (e.g. sensing surface of an optical CCD or CMOS detector) to provide a maximum signal at the predetermined detection location. The signal at the predetermined detection location is compared with a reference signal which may be, for example, the signal of the light diffracted at the binding sites only (without target molecules bound thereto) or with another (known) reference signal. Alternatively, a time sensitive measurement can be carried out to measure the signal representative of the binding affinity which is to be compared to an ealier measured signal representative of the binding affinity. The propagation of non-diffracted portions of the incident beam of coherent light to the predetermined detection location is prevented if the non-diffracted portion is either completely masked out or if the intensity of the non-diffracted portion is greatly reduced. The reduction needs to be such that the signal at the predetermined detection location is not adversely affected by the non-diffracted portions of the coherent light so that an interpretation of the signal is possible. Technically, the term "non-diffracted portions" may be interpreted to include all portions of the incident beam of coherent light which are different from the diffracted portion. Particularly, the non-diffracted portions may comprise reflected portions (reflected at a surface inside or outside the substrate), refracted portions (refracted at an interface between the substrate and the surrounding medium) or portions (parts) of the incident beam which directly propagate to the predetermined detection location.

Since the diffracted light is of low intensity compared with the intensity of non-diffracted light, e.g. light which is reflected or refracted at the substrate, the propagation of the non-diffracted portions of the incident beam of light to the predetermined detection location needs to be prevented by the beam stop. Advantageously, the detected intensity signal originating from the target molecules bound to the binding sites arranged along these lines increases with the square of the quantity of bound target molecules compared with the signal originating from unspecifically bound (randomly arranged and not along the curved lines arranged) target molecules for which the detected intensity signal only linearly increases with the quantity of unspecifically bound target molecules. In principle, this renders obsolete (or at least reduces the need for) washing away any unspecifically bound target molecules from the planar surface prior to detection of the signal originating from specific binding of target molecules to binding sites on the planar surface.

The diffracted portions from different locations on the same line as well as from locations on different lines contribute to the maximum signal at the predetermined detection location as long as the condition that the difference in optical path length at the predetermined detection location is a multiple integer of the predetermined wavelength is fulfilled for the different portions. This can be achieved by lines which are arranged as a phase grating that forms a diffractive lens which focusses the diffracted portions to the predetermined detection location in a diffracting manner so that the diffracted portions interfere at the focal location (predetermined detection location), i.e. with curved lines having a graded distance from each other. The intensity of the signal at the predetermined detection location increases, *inter alia,* with the number of lines (assuming that there is a constant density (number per surface area) of diffraction centers (molecules) along the curved lines) at which a portion of the incident beam of coherent light is diffracted by the target molecules bound to the binding sites. The distance between adjacent lines varies and ranges in a particular example from 260 nm to 680 nm. Lines within the meaning of the present invention are ideal lines which define the locations at the planar surface where the binding sites are arranged. Deviations (in particular random deviations) of the arrangement of the diffraction centers (molecules) from the ideal lines, i.e. variations of the distance of the diffraction centers from the ideal lines, do not deteriorate the signal at the predetermined detection location, as long as the majority of such deviations are smaller than a quarter of the distance between adjacent lines. The optical path length is the product of the predetermined wavelength of the coherent light and the refractive index of the material through which the coherent light propagates, e.g. air, sample solution ($n_{aqueous}$ solution $\approx 1.33$) or a liquid immersion ($n_{immersion} \approx n_{substrate}$) or the substrate ($n_{glass} = 1.521$), respectively. In the best case, the diffracted portions interfere at the predetermined detection location having a spatially distributed intensity profile of an Airy disk (i.e. the focused spot of light that an "ideal" lens with a circular aperture produces limited by the diffraction of the light) having a diameter given by Abbe's formula $D = \lambda /2NA$, wherein D is the diameter of the (circular) area covered by the curved lines and NA is the numerical aperture which is defined technically analog to an aperture of a microscope.

Preferably, the substrate comprises a material which is transparent to the coherent light of the predetermined wavelength so as to allow for propagation of the incident beam of coherent light and of the diffracted portion of coherent light through the substrate. This allows for an advantageous use of the device in which the incident beam of light is directed through the substrate to the curved lines and is diffracted therefrom, again through the substrate, to the predetermined detection location. In a particular example, the complete substrate is made of the transparent material.

Advantageously, the beam stop comprises a non-transparent section which is arranged on or within the substrate in a manner to prevent propagation of the non-diffracted portions (e.g. reflected portions) of the incident beam of coherent light to the predetermined detection location. The non-transparent section can be a section of the substrate which is made of a light absorbing material, or can be a separate element arranged in or on the substrate.

According to one aspect, the beam stop comprises an anti-reflective section arranged at the planar surface of the substrate. This allows for preventing the non-diffracted portions (e.g. reflected portions) of the incident beam of coherent light from propagating to and from impinging on the detection location. In one particular example, the anti-reflective layer is an optical coating on the planar surface having a thickness and being made of a material so as to be capable of reducing or preventing non-diffracted portions (reflected portions) of light from propagating to the predetermined detection location.

In a further embodiment of the invention, the beam stop comprises a deflector body arranged on or within the substrate. The deflector body has a curved outer surface which is capable of in operation scattering off the coherent light incident on the deflector body in a manner to prevent propagation of the non-diffracted portion (e.g. reflected portion) of the incident beam of coherent light to the predetermined detection location. In other words, the non-diffracted portions of the incident beam of coherent light are dissipated. Dissipation in this regard denotes the reduction of the intensity of the non-diffracted portion (e.g. reflected portion) of coherent light impinging on the detection location to an extent such that the diffracted portion of the incident beam of coherent light can be detected to generate a signal representative of the binding affinity. The deflector body may be embodied as a metallic sphere and can be arranged in the substrate adjacent to the planar surface. The metallic sphere may have a curved outer metal surface which is capable of deflecting the incident beam of coherent light over a wide angle in a dissipating manner so that the deflected light propagates in a variety of directions with reduced intensity compared with the intensity of non-deflected light of the non-diffracted portion (reflected portion) which propagates in only one direction.

According to one aspect, the predetermined beam generation location and the predetermined detection location are arranged on (or close to) an outer surface of the substrate opposite to the planar surface of the substrate. The predetermined beam generation location and the predetermined detection location arranged on (or close to) the outer surface opposite to the planar surface of the substrate are arranged in a common plane which is parallel to the planar surface of the substrate. The predetermined beam generation location can be arranged at the outer surface opposite to the planar surface of the substrate by attaching a laser light source thereto which generates a laser light beam propagating as an incident beam of coherent light. The predetermined detection location can also be arranged at the outer surface opposite to the planar surface of the substrate by attaching a CCD or CMOS detector to the outer surface opposite to the planar surface of the sbustrate.

The device comprises a carrier having the predetermined beam generation location and the predetermined detection location arranged thereon. The carrier is arranged with respect to the substrate such that the predetermined beam generation location and the predetermined detection location are arranged in a common plane which is parallel to the planar surface of the substrate. Depending on the size and the geometry of the plurality of curved lines, it may be of advantage to arrange the beam generation location and the detection location on the carrier. This allows for variation of the distance between of the predetermined detection location and the lines arranged on the substrate. This is particularly advantageous in case of differences in the dimensions of the device, e.g. dimensional differences caused by manufacturing tolerances.

Each line of the plurality of curved lines is arranged on the planar surface of the substrate such that the total optical path length of the incident coherent light from the predetermined beam generation location to the binding sites arranged on the respective same curved line and the optical path length and of the diffracted portion of the coherent light from the respective same curved line to the predetermined detection location is constant. For the predetermined beam generation location and the predetermined detection location which are arranged separated in a plane parallel to the planar surface of the substrate, the constant distance results in curved lines having an elliptic geometry. In a particular example, the curved lines are geometrically defined in an x,y-plane at the planar surface of the substrate by the following equation.

$$y_j{}^2 = \left(\frac{(j_0+j)\,\lambda}{2\,n_s}\right)^2 + \left(\frac{2\,n_s\;\xi\;x_j}{(j_0+j)\,\lambda}\right)^2 - \left(f^2+\xi^2+x_j{}^2\right)$$

wherein

$j_0$ , $j$    is an integer,
$\lambda$        is the predetermined vacuum wavelength of the coherent light,

$n_s$     is the refractive index of the substrate,

$\xi$     is half distance between the beam generation location and the detection location and

f     is a number which corresponds to the distance between the center of the curved lines and the detection location.

Adjacent curved lines of the plurality of curved lines are arranged spaced from one another by a distance such that the total optical path length of the incident coherent light from the predetermined beam generation location to the binding sites arranged on different curved lines of the plurality of curved lines and of the diffracted portions of the coherent light from the binding sites arranged on these different curved lines to the predetermined detection location has a difference which is a multiple integer of the predetermined wavelength of the coherent light. This causes the light from the predetermined beam generation location and diffracted towards the predetermined detection location to constructively interfere at the detection location to provide a maximum signal.

According to one aspect, the predetermined detection location is a sensing surface of a CCD or CMOS detector having a plurality of pixels arranged on the sensing surface in a grid-like manner. Each pixel has a size so as to be capable of detecting less than one half of the complete spatially distributed intensity of the interfering diffracted portions of the coherent light at the predetermined detection location in one single pixel . The sensing surface comprises a two-dimensional grid of pixels which allows for detection of the intensity of the interfering diffracted portions of the incident beam of coherent light. In a specific example, the pixel size is smaller than 1 $\mu m^2$ (square micrometer) to allow for detecting the focus in a single pixel without any background signal.

According to one aspect, the device further comprises a point light source arranged at the predetermined beam generation location and capable of generating a divergent incident beam of coherent light. The point light source may be embodied as a laser beam propagating through a small aperture to cause an emanating spherical wave.

According to a further aspect, a separate outer layer is provided on the planar surface of the substrate. The outer layer may be an outer compact layer or an outer porous layer. An outer porous layer may comprise a material having a porosity such that only (or preferentially) target molecules may penetrate through the outer porous layer. The outer compact layer or outer porous layer can be arranged directly on the planar surface or at a small distance therefrom so as to form a channel through which a fluid may flow (e.g. a fluid capable of transporting the target molecules or a fluid capable of transporting complementary binder molecules which in turn may be capable of binding to the target molecule and which may carry a scattering enhancer).

According to a further aspect of the invention, a method for detecting binding affinities is according to appended claim 11.

Preferably, the method according to the invention before applying the plurality of target molecules to the binding sites comprises the steps of:

- at the predetermined beam generation location, generating the incident beam of coherent light which is to be diffracted at the binding sites only in a manner such that diffracted portions of the incident beam of coherent light interfere at the predetermined detection location with a difference in optical path length which is a multiple integer of the predetermined wavelength of the coherent light to provide a signal representative of the binding sites only at the predetermined detection location; and
- at the predetermined detection location, measuring the signal representative of the binding sites only to provide the reference signal.

Preferably, the method further comprises the step of applying a plurality of complementary binder molecules to the planar surface of the substrate , with the complementary binder molecule being capable of binding to the target molecules bound to the binding site, and with the complementary binder molecules comprising a scattering enhancer. In a particular example, the scattering enhancer comprises gold (gold nano-particle) and has a size of several nanometers, in particular in the range of 10 nm to 150 nm. A change in size of the scattering enhancer may be capable of changing the sensitivity of the detection of binding affinities. In one example, multiple complementary binder molecules can bind to a single scattering enhancer to allow for multiple bond interactions showing a combined strength (avidity).

Further advantageous aspects of the invention become apparent from the following description of embodiments of the invention with reference to the accompanying drawings in which:

Fig. 1     shows a plan view from above of a device according to an embodiment of the invention having a plurality of elliptically curved lines and a deflector body as beam stop;

Fig. 2     shows a side view on the device of Fig. 1 and illustrates the optical paths of the incident beam of light as well as of the diffracted and reflected portions thereof;

Fig. 3     shows the side view of the device of Fig. 1 having a carrier, wherein the incident beam of coherent light is divergent;

Fig. 4      shows the device of Fig. 3 having a porous layer arranged above the planar surface;

Fig. 5      shows another embodiment of a device according to the invention having a non-transparent section as beam stop at an outer surface opposite to the planar surface of the substrate, wherein the incident beam of coherent light is parallel;

Fig. 6      shows a side view on the device of Fig. 1 and a variant of the arrangement of the beam generation location and the detection location relative to the device;

Figs. 7a-7b      show a top view on the device of Fig. 1 having arranged thereon the plurality of lines in a predetermined surface area arranged relative to the beam generation location and the detection location to allow for total reflection of the coherent light;

Fig. 8      shows a side view of the device of Fig. 7a;

Figs. 9-12      show steps of a sandwich-assay carried out for the detection of binding affinities; and

Fig. 13      shows a variant of the sandwich-assay of Figs. 9-12 using a different scattering enhancer.

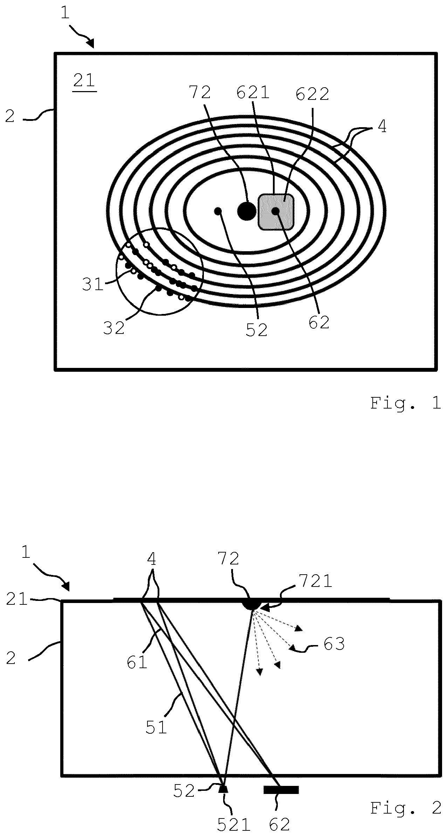

**Fig. 1** shows an embodiment of a device 1 according to the invention. Structurally, one plurality of curved lines 4 is arranged at a planar surface 21 of a transparent substrate 2. In an enlarged view within the circle shown in Fig. 1, binding sites 31 and target molecules 32 are illustrated. Because the density of the curved lines is high, only each fiftieth individual curved line 4 is shown in Fig. 1 together with some adjacent lines. In reality, also the spaces between the groups of curved lines 4 shown in Fig. 1 are filled with curved lines. Curved lines 4 in Fig. 1 are arranged to define ellipses. The elliptical arrangement is such that the integrated optical path length of coherent light 51 from a beam generation location 52 to a specific curved line 4 and the optical path length of a diffracted portion 61 of the coherent light from the specific curved line 4 to a detection location 62 are constant for each line. Curved lines 4 are arranged at an adjacent distance such that the integrated optical path length of coherent light from beam generation location 52 to different curved lines 4 and of the diffracted portion 61 of the coherent light from the respective different curved lines 4 to detection location 62 has a difference which is a multiple integer of the predetermined wavelength of the coherent light 63. In the present example, the distance between adjacent curved lines is in the range of about 300 nm to 600 nm for a wavelength of the coherent light of 635 nm. Beam generation location 52 and a sensing surface (not shown) of CCD or CMOS detector 621 forming detection location 62 are arranged in a common plane parallel to planar surface 21 in a predetermined distance below device 1. A beam stop is arranged inside substrate 2. The beam stop is a deflector body 72 arranged inside substrate 2 at planar surface 21 and is arranged between beam generation location 52 and detection location 62 along the path of propagation of the coherent light .

The use of the device of Fig. 1 in the detection of binding affinities and the arrangement of curved lines 4 are described with reference to **Fig. 2.**

In use, incident beam of coherent light 51 is generated at beam generation location 52 (e. g. a laser as point light source 521 (a spot light source) from which a divergent incident beam of coherent light emanates). Light emanating from beam generation location 52 impinges on curved lines 4 (the lines are not shown as separate lines in the present view). Incident beam 51 is illustrated to propagate towards two different curved lines 4 of the plurality of curved lines 4. After being diffracted at binding sites 31 bound to target molecule 32, diffracted portions 61 of the coherent light impinge on detection location 62. Impinging portions 61 of light diffracted at different lines at the detection location 62 have a difference in the optical path length (beam generation location - respective line - detection location) which is a multiple integer of the wavelength of the coherent light and contribute to the maximum intensity signal at the detection location 62.

Curved lines 4 are arranged such that the integrated (total) optical path length of the coherent light 51 from beam generation location 52 to the different curved lines 4 (not separately shown) and of the diffracted portion 61 from respective different curved lines 4 to the detection location 62 is a multiple integer of the predetermined wavelength of the coherent light. Hence, for different lines 4 the respective integrated optical path length has a difference which is a multiple integer of the wavelength of the coherent light.

To prevent reflected portions 63 (representing non-diffracted portions) of the incident beam of coherent light 51 to propagate to the detection location 62, a half sphere (or a polygon) is arranged within substrate 2 as deflector body 72 proximate to the planar surface 21 of the substrate. Deflector body 72 has an outer surface 721 of convex shape so as to be capable of scattering off incident coherent light in different directions. This reduces the intensity of light reflected by the deflector body 72 in the direction of the detection location. Hence, deflector body 72 makes sure that reflected light 63 does not impinge on detection location 62, or that the intensity of reflected light impinging on detection location is at least greatly reduced. Deflector body 72 is arranged at a position at which it eliminates (or reduces) the reflection of incident beam 51 at the planar surface 21 towards detection location 62.

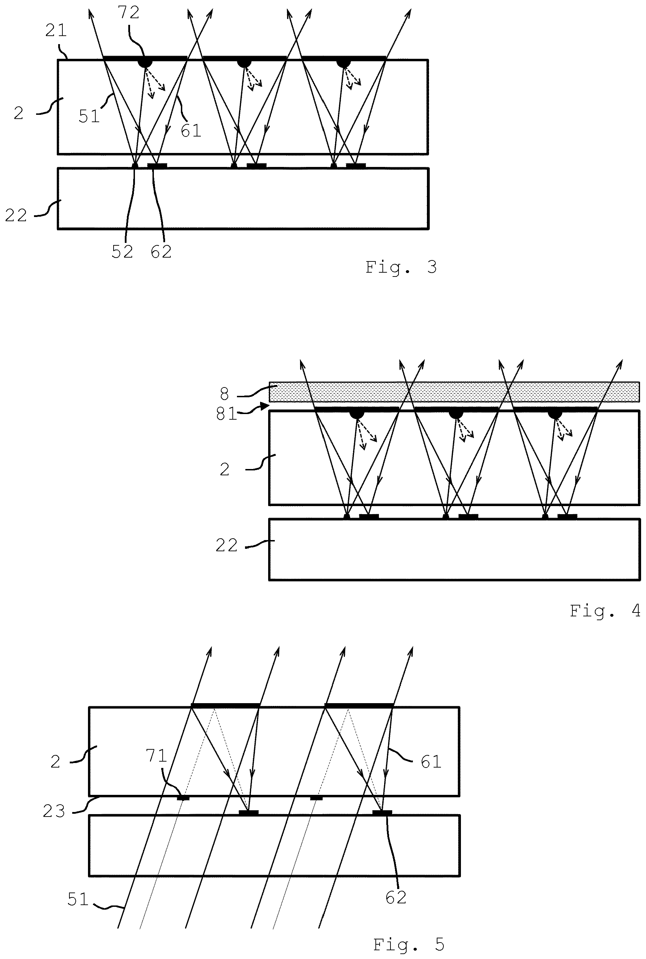

In **Fig. 3,** a divergent incident beam of coherent light 51 is generated at beam generation location 52 which is arranged on a carrier 22. Carrier 22 may be a further planar substrate havingarranged thereon a laser light source, for example. The coherent light emitted from the lase light source is diffracted so that diffracted portion 61 impinges on detection location 62 arranged on carrier 22. Beam generation location 52 and detection location 62 are both arranged on carrier 22 so as to be arranged in a common plane parallel to planar surface 21 of the substrate 2.

Another variant is shown in **Fig. 4** which is in principle similar to the device shown in Fig. 3, however, it has arranged thereon a porous layer 8. Porous layer 8 is arranged at a distance to planar surface 21 to form a channel 81 which allows for transporting the binding sites, the target molecule or a complementary binder molecule (not shown) in a fluid through the channel 81 for applying them to planar surface 21.

In **Fig. 5** another embodiment of a device 1 is shown having a non-transparent section 71 forming the beam stop. Non-transparent section 71 is arranged at an outer surface 23 opposite to planar surface 21 of substrate 2. A parallel incident beam of coherent light 51 is diffracted at the curved lines (not shown) so that diffracted coherent light 61 impinges on detection location 62. A part of the parallel incident beam of coherent light 51 which would form a reflected portion (non-diffracted portion) impinging on detection location 62 is masked out by non-transparent section 71.

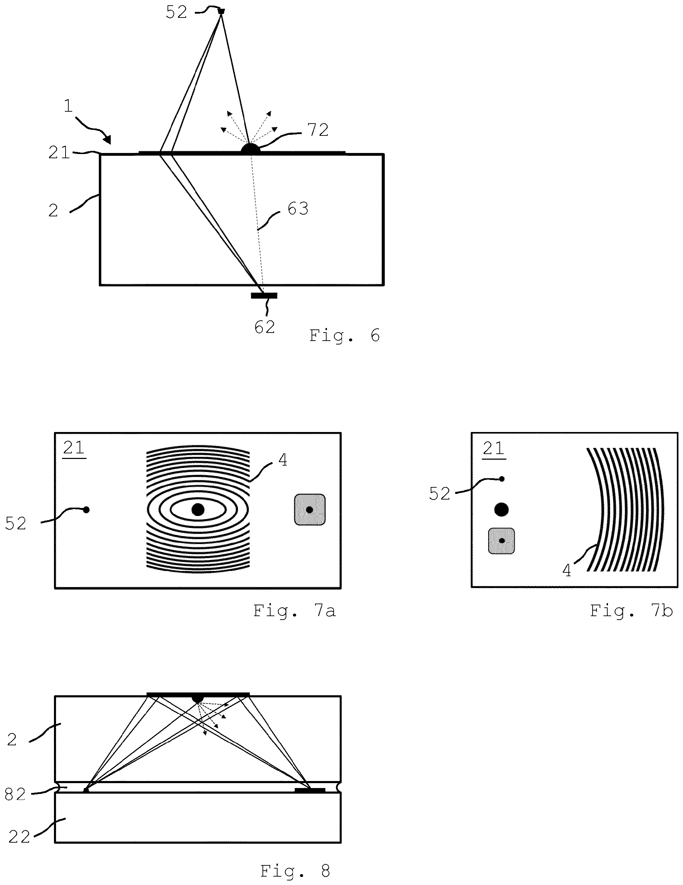

**Fig. 6** is a side view of the device as it has in principle been already explained with reference to Fig. 2. However, different therefrom the beam generation location 52 and the detection location 62 are arranged at opposite sides of device 1 (beam generation 52 is arranged above device 1 and detection location 62 is arranged below device 1). In this example, the beam stop is a deflector body 72 arranged at the planar surface 21 so as to prevent non-diffracted portions 63 (refracted portions) of the incident beam of coherent light from propagating to the detection location 62. The refracted portion 63 is in the shown example the part of the incident beam of light which changes the direction of propagation so as to propagate to the detection location when passing the interface between the substrate and the medium surrounding the substrate.

Both **Fig. 7a** and **Fig. 7b** relate to another aspect according to which the beam generation location is arranged with respect to the plurality of curved lines 4 on the planar surface 21 such that the incident beams of coherent light impinge on the plurality of curved lines 4 under an angle of total reflection, in particular "total internal reflection". Hence, no light of the totally reflected incident beam of coherent light propagates out of the substrate through the interface between the substrate and the medium above the planar surface 21. Actually, the totally reflected light penetrates through the outer surface a predetermined distance in order to be diffracted at the plurality of curved lines 4. For total reflection the predetermined distance is given by the penetration depth of the evanescent field at the point of total internal reflection.

Furthermore, Fig. 7a and Fig. 7b, relate to another aspect according to which the detection location 62 is arranged on the planar surface 21 such that the diffracted rays of the beam of coherent light emanate (i.e. interfere outgoing) from the curved lines 4 under an angle relative to the normal of the planar surface 21 which is larger than the critical angle of total internal reflection; the latter angle depends on the refractive indices of the substrate and the medium above the substrate. Hence, no light from above the planar surface 21 reaches the detection location through the region covering the plurality of curved lines 4 on the planar surface 21. This aspect of the invention increases the detection sensitivity of the device in use.

As can be seen in **Fig. 8** which is the side view of the device the planar surface of which is shown in Fig. 7a, total reflection of the incident beam of coherent light is achieved by arranging the beam generation location relative to the plurality of adjacently arranged curved lines such that the incident light impinges under an angle for which total reflection occurs (an angle larger than a particular critical angle with respect to the normal to the surface, which critical angle depends on the refractive indices of the substrate and the medium above the substrate). Between substrate 2 and carrier 22, an immersion liquid 82 of a predetermined refractive index (e.g. the same as that of the substrate and/or the carrier) is provided. As shown in Fig. 8, diffracted beams of coherent light emanate from the plurality of curved lines 4 under an angle to the normal of the planar surface 21 which is larger than the critical angle of total (internal) reflection.

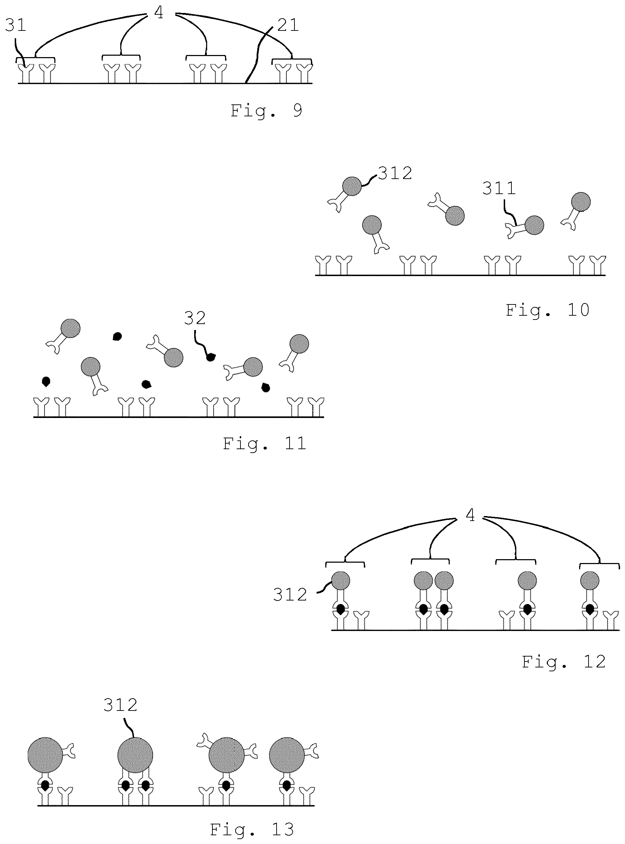

Figs. 9-12 illustrate four steps of a sandwich-assay carried out on a planar surface 21 of a device according to an embodiment of the invention. Such a "sandwich" comprises a binding site 31 bound to planar surface 21, a target molecule 32 bound to binding site 31 and a complementary binder molecule 311 bound to target molecules 32. In a first step **(Fig. 9)** binding sites 31 are arranged on planar surface 21 of such a device along a plurality of curved lines 4.

Complementary binder molecule 311 shown in **Fig. 10** has a scattering enhancer 312 which is in a particular example a gold-nano particle capable of increasing the intensity of a diffracted portion 61 of coherent light. Complementary binder molecules 311 are randomly disposed in the proximity of the binding sites, e.g. by spraying them onto planar surface 21 or by attaching them to the porous layer (see Fig. 4 and Fig. 8). Randomly disposed scattering enhancers 312 provide no interference maximum at the detection location but only generate scattered background light.

Target molecules 32 are applied to binding sites 31 **(Fig. 11)** so that at such binding site 31, target molecule 32 and complementary binder molecule 311 comprising scattering enhancer 312 form a "sandwich". Hence, scattering enhancer 312 is arranged along a plurality of curved lines 4 (**Fig. 12**). The signal provided by light diffracted by scattering enhancers 312 arranged along the plurality of curved lines 4 is of a high intensity compared with the signal provided by the remaining randomly arranged scattering enhancers 312.

Illustrated in **Fig. 13** is a variant of the sandwich-assay shown in Figs. 9-12. According to a first aspect, the scattering enhancer 312 provided at the complementary binder molecules is larger in size (increased size compared to Figs. 9-12) so as to generate a higher intensity of the diffracted portion to increase sensitivity of the device in use. According to a second aspect, the scattering enhancer 312 binds to more than one (e.g. two, three, etc.) complementary binder molecules. Two complementary binder molecules allow for binding to two different target molecules simultaneously or subsequently (within short periods of time) to allow for a multiple bond interaction having a combined strength (avidity).

## Claims

1.  Device (1) for use in the detection of binding affinities, the device (1) comprising a substrate (2) devoid of a waveguide, the substrate (2) comprising a planar surface (21) having arranged thereon a plurality of binding sites (31) capable of binding with a target molecule (32), the device further comprising a predetermined beam generation location (52), a predetermined detection location (62) and a beam stop (7) which is arranged on the surface of the substrate, to, in operation, prevent propagation of non-diffracted portions (63) of a beam of coherent light (51) of a predetermined wavelength generated at the beam generation location (52) and incident on the binding sites (31) with the bound target molecule (32) to the predetermined detection location (62), **characterized in that** the binding sites (31) are arranged on the planar surface (21) along a plurality of adjacently arranged curved lines (4) which are spaced from one another by a distance to, in operation, cause the beam of coherent light (51) of the predetermined wavelength generated at the predetermined beam generation location (52) relative to the plurality of adjacently arranged curved lines (4) and incident on the binding sites (31) with the bound target molecule (32) to be diffracted at the binding sites (31) with the bound target molecule (32) in a manner such that diffracted portions (61) of the incident beam of coherent light (51) interfere at the predetermined detection location (62) relative to the plurality of adjacently arranged curved lines (4) with a difference in optical path length which is a multiple integer of the predetermined wavelength of the coherent light to provide a signal representative of the binding affinity of the binding sites (31) and the target molecule (32), **in that** the device further comprises a carrier (22) having the predetermined beam generation location (52) and the predetermined detection location (62) arranged thereon, the carrier (22) being arranged with respect to the substrate (2) such that the predetermined beam generation location (52) and the predetermined detection location are arranged in a common plane which is parallel to the planar surface (21) of the substrate (2), and **in that** each line of the plurality of curved lines (4) is arranged on the planar surface (21) of the substrate (2) such that the total path length of the incident coherent light (51) from the predetermined beam generation location (52) to the binding sites (31) arranged on the respective same curved line (4) plus the diffracted portion (61) of the coherent light from the binding sites on the same curved line (4) to the predetermined detection location is constant for all binding sites on the same curved line (4), with the curved lines being geometrically defined in an x,y-plane at the planar surface of the substrate by the following equation:

$$y_j{}^2 = \left(\frac{(j_0+j)\,\lambda}{2\,n_s}\right)^2 + \left(\frac{2\,n_s\;\xi\;x_j}{(j_0+j)\,\lambda}\right)^2 - \left(f^2+\xi^2+x_j{}^2\right)$$

    wherein

        $j_0$, $j$ is an integer,
        $\lambda$ is the predetermined vacuum wavelength of the coherent light,
        $n_s$ is the refractive index of the substrate,
        $\xi$ is half distance between the beam generation location and the detection location and is non-zero, and
        f is a number which corresponds to the distance between the center of the curved lines and the detection location.

2.  Device (1) according to claim 1, wherein the substrate (2) comprises a material which is transparent to the coherent light of the predetermined wavelength so as to allow for propagation of the incident beam of coherent light (51) and of the diffracted portions (61) of coherent light through the substrate (2).

3.  Device (1) according to claim 2, wherein the beam stop (7) comprises an non-transparent section (71) which is arranged on or within the substrate (2) in a manner to prevent propagation of the non-diffracted portions (63) of the incident beam of coherent light (51) to the predetermined detection location (62).

4. Device (1) according to claim 2, wherein the beam stop (7) comprises an anti-reflective section arranged at the planar surface (21) of the substrate (2).

5. Device (1) according to claim 2, wherein the beam stop (7) comprises a deflector body (72) arranged on or within the substrate (2), the deflector body (72) having a curved outer surface (721) which is capable of in operation scattering off the coherent light incident on the deflector body (72) in a manner to prevent propagation of the non-diffracted portion (63) of the incident beam of coherent light to the predetermined detection location (62).

6. Device (1) according to anyone of the preceding claims, wherein the predetermined beam generation location (52) and the predetermined detection location (62) are arranged on an outer surface (23) of the substrate (2) opposite to the planar surface (21), wherein the predetermined beam generation location (52) and the predetermined detection location (62) are arranged on the outer surface (23) opposite to the planar surface (21) of the substrate (2) in a common plane which is parallel to the planar surface (21) of the substrate (2).

7. Device (1) according to claim 1, wherein adjacent curved lines (4) of the plurality of curved lines (4) are arranged spaced from one another by a distance such that the total optical path length of the incident coherent light (51) from the predetermined beam generation location (52) to the binding sites (31) arranged on different curved lines (4) and of the diffracted portions (61) of the coherent light from the binding sites arranged on these different curved lines (4) to the predetermined detection location (62) has a difference which is a multiple integer of the predetermined wavelength of the coherent light.

8. Device (1) according to anyone of the preceding claims, wherein the predetermined detection location (62) is a sensing surface (622) of a CCD or CMOS detector (621) having a plurality of pixels arranged on the sensing surface (622) in a grid-like manner, each pixel having a size so as to be capable of detecting less than one half of the complete spatially distributed intensity of the interfering diffracted portions (61) of the coherent light at the predetermined detection location (62) in one single pixel.

9. Device (1) according to anyone of the preceding claims, further comprising a point light source (521) arranged at the beam generation location (52) and capable of generating a divergent incident beam of coherent light (51).

10. Device (1) according to anyone of the preceding claims, further comprising a separate outer layer (8) provided on the planar surface (21) of the substrate (2).

11. Method for detecting binding affinities comprising the steps of:

- providing a device (1) according to anyone of the preceding claims;
- applying a plurality of target molecules (32) to the binding sites (31);
- at a predetermined beam generation location (52) relative to the plurality of adjacently arranged curved lines (4) generating a beam of coherent light (51) of a predetermined wavelength and incident on the binding sites (31) with the bound target molecule (32) so as to be diffracted at the binding sites (31) with the target molecule (32) bound thereto in a manner such that diffracted portions (61) of the incident beam of coherent light (51) interfere at a predetermined detection location (62) relative to the plurality of adjacently arranged curved lines (4) with a difference in optical path length which is a multiple integer of the predetermined wavelength of the coherent light (51) to provide a signal representative of the binding sites (31) with the target molecule (32) bound thereto at the predetermined detection location (62);
- at the predetermined detection location (62) measuring the signal representative of the binding sites (31) with the target molecule (32) bound thereto; and
- detecting the binding affinity of the binding sites (31) and the target molecule (32) by comparing the signal representative of the binding sites (31) with the target molecule (32) bound thereto with a reference signal representative of the binding sites (31) only.

12. Method according to claim 11, the method before applying the plurality of target molecules (32) to the binding sites (31) comprising the steps of:

- at the predetermined beam generation location (52) generating the incident beam of coherent light (51) which is to be diffracted at the binding sites (31) only in a manner such that diffracted portions (61) of the incident beam of coherent light (51) interfere at the predetermined detection location (62) with a difference in optical path length which is a multiple integer of the predetermined wavelength of the coherent light (51) to provide a signal representative of the binding sites (31) only at the predetermined detection location (62); and

- at the predetermined detection location (62) measuring the signal representative of the binding sites (31) only to provide the reference signal.

13. Method according to anyone of claims 11 or 12, further comprising the step of:

- applying a plurality of complementary binder molecules (311) to the planar surface (21) of the substrate, the complementary binder molecules (311) being capable of binding to the target molecules (32) bound to the binding sites (31), wherein the complementary binder molecules (311) comprise a scattering enhancer (312).

**Patentansprüche**

1. Vorrichtung (1) zur Verwendung bei der Erkennung von Bindungsaffinitäten, wobei die Vorrichtung (1) ein Substrat (2) ohne einen Wellenleiter umfasst, wobei das Substrat (2) eine ebene Oberfläche (21) umfasst, auf der eine Vielzahl von Bindungsstellen (31) angeordnet sind, die in der Lage sind, eine Bindung mit einem Zielmolekül (32) einzugehen, wobei die Vorrichtung überdies einen vorbestimmten Strahlerzeugungsort (52), einen vorbestimmten Detektionsort (62) und einen Strahlstopp (7) umfasst, der auf der Oberfläche des Substrats angeordnet ist, um im Betrieb die Ausbreitung von nicht gebeugten Anteilen (63) eines kohärenten Lichtstrahls (51) einer vorbestimmten Wellenlänge, der an dem Strahlerzeugungsort (52) erzeugt wird und auf die Bindungsstellen (31) mit dem gebundenen Zielmolekül (32) einfällt, zu dem vorbestimmten Detektionsort (62) zu verhindern, **dadurch gekennzeichnet, dass** die Bindungsstellen (31) auf der ebenen Oberfläche (21) entlang einer Vielzahl von benachbart angeordneten gekrümmten Linien (4) angeordnet sind, die in einem Abstand voneinander angeordnet sind, um im Betrieb zu verursachen, dass der kohärente Lichtstrahl (51) der vorbestimmten Wellenlänge, der an dem vorbestimmten Strahlerzeugungsort (52) im Verhältnis zu der Vielzahl von benachbart angeordneten gekrümmten Linien (4) erzeugt wird und auf die Bindungsstellen (31) mit dem gebundenen Zielmolekül (32) einfällt, an den Bindungsstellen (31) mit dem gebundenen Zielmolekül (32) gebeugt wird, derart, dass gebeugte Anteile (61) des einfallenden kohärenten Lichtstrahls (51) an dem vorbestimmten Detektionsort (62) relativ zu der Vielzahl von benachbart angeordneten gekrümmten Linien (4) mit einer Differenz in der optischen Weglänge interferiert, die ein ganzzahliges Vielfaches der vorbestimmten Wellenlänge des kohärenten Lichts beträgt, um ein Signal bereit zu stellen, das repräsentativ für die Bindungsaffinität der Bindungsstellen (31) und des Zielmoleküls (32) ist, **dadurch, dass** die Vorrichtung überdies einen Träger (22) umfasst, auf dem der vorbestimmte Strahlerzeugungsort (52) und der vorbestimmte Detektionsort (62) angeordnet sind, wobei der Träger (22) in Bezug zum Substrat (2) so angeordnet ist, dass der vorbestimmte Strahlerzeugungsort (52) und der vorbestimmte Detektionsort in einer gemeinsamen Ebene angeordnet sind, die parallel zur ebenen Oberfläche (21) des Substrats (2) ist **und dadurch, dass** jede Linie der Vielzahl von gekrümmten Linien (4) auf der ebenen Oberfläche (21) des Substrats (2) so angeordnet ist, dass die gesamte Weglänge des einfallenden kohärenten Lichts (51) von dem vorbestimmten Strahlerzeugungsort (52) bis zu den Bindungsstellen (31), die auf der jeweils gleichen gekrümmten Linie (4) angeordnet sind, plus dem gebeugten Anteil (61) des kohärenten Lichts von den Bindungsstellen auf der gleichen gekrümmten Linie (4) bis zum vorbestimmten Detektionsort für alle Bindungsstellen auf der gleichen gekrümmten Linie (4) konstant ist, wobei die gekrümmten Linien dabei in einer x,y-Ebene an der ebenen Oberfläche des Substrats mittels der folgenden Gleichung geometrisch definiert sind:

$$y_j^2 = \left(\frac{(j_0+j)\,\lambda}{2n_s}\right)^2 + \left(\frac{2\,n_s\,\,\xi\,\,x_j}{(j_0+j)\,\lambda}\right)^2 - \left(f^2 + \xi^2 + x_j^2\right)$$

wobei

$j_0$, $j$ eine ganze Zahl ist,
$\lambda$ die vorbestimmte Vakuum-Wellenlänge des kohärenten Lichts ist,
$n_s$ der Brechungsindex des Substrats ist,
$\zeta$ der halbe Abstand zwischen dem Strahlerzeugungsort und dem Detektionsort ist und ungleich Null ist, und
$f$ eine Zahl ist, die dem Abstand zwischen dem Mittelpunkt der gekrümmten Linien und dem Detektionsort entspricht.

**2.** Vorrichtung (1) nach Anspruch 1, wobei das Substrat (2) ein Material umfasst, das gegenüber dem kohärenten Licht der vorbestimmten Wellenlänge transparent ist, um eine Ausbreitung des einfallenden kohärente Lichtstrahls (51) und der gebeugten Anteile (61) des kohärenten Lichts durch das Substrat (2) hindurch zuzulassen.

**3.** Vorrichtung (1) nach Anspruch 2, wobei der Strahlstopp (7) einen nicht transparenten Abschnitt (71) umfasst, der auf dem oder innerhalb des Substrats (2) auf eine Weise angeordnet ist, um eine Ausbreitung der nicht gebeugten Anteile (63) des einfallenden kohärenten Lichtstrahls (51) zum vorbestimmten Detektionsort (62) zu verhindern.

**4.** Vorrichtung (1) nach Anspruch 2, wobei der Strahlstopp (7) einen reflexmindernden Abschnitt umfasst, der an der ebenen Oberfläche (21) des Substrats (2) angeordnet ist.

**5.** Vorrichtung (1) nach Anspruch 2, wobei der Strahlstopp (7) einen Deflektorkörper (72) umfasst, der auf oder innerhalb des Substrats (2) angeordnet ist, wobei der Deflektorkörper (72) eine gekrümmte äussere Oberfläche (721) aufweist, die im Betrieb das kohärente Licht, das auf den Deflektorkörper (72) einfällt, auf eine Weise streuen kann, um eine Ausbreitung des nicht gebeugten Anteils (63) des einfallenden kohärenten Lichtstrahls zum vorbestimmten Detektionsort (62) zu verhindern.

**6.** Vorrichtung (1) nach einem der vorangehenden Ansprüche, wobei der vorbestimmte Strahlerzeugungsort (52) und der vorbestimmte Detektionsort (62) auf einer äußeren Oberfläche (23) des Substrats (2) gegenüber der ebenen Oberfläche (21) angeordnet sind, wobei der vorbestimmte Strahlerzeugungsort (52) und der vorbestimmte Detektionsort (62) auf der äusseren Oberfläche (23) gegenüber der ebenen Oberfläche (21) des Substrats (2) in einer gemeinsamen Ebene angeordnet sind, die parallel zu der ebenen Oberfläche (21) des Substrats (2) ist.

**7.** Vorrichtung (1) nach Anspruch 1, wobei benachbarte gekrümmte Linien (4) der Vielzahl von gekrümmten Linien (4) so in einem Abstand voneinander angeordnet sind, dass die gesamte optische Weglänge des einfallenden kohärenten Lichts (51) von dem vorbestimmten Strahlerzeugungsort (52) zu den Bindungsstellen (31), die auf verschiedenen gekrümmten Linien (4) angeordnet sind, und der gebeugten Anteile (61) des kohärenten Lichts von den Bindungsstellen, die auf diesen verschiedenen gekrümmten Linien (4) angeordnet sind, zum vorbestimmten Detektionsort (62), eine Differenz aufweist, die ein ganzzahliges Vielfaches der vorbestimmten Wellenlänge des kohärenten Lichts ist.

**8.** Vorrichtung (1) nach einem der vorangehenden Ansprüche, wobei der vorbestimmte Detektionsort (62) eine Sensoroberfläche (622) eines CCD-Detektors oder CMOS-Detektors (621) ist, der eine Vielzahl von Pixeln aufweist, die gitternetzartig auf der Sensoroberfläche (622) angeordnet sind, wobei jeder Pixel eine Grösse aufweist, um in der Lage zu sein, weniger als die Hälfte der gesamten räumlich verteilten Intensität der interferierenden gebeugten Anteile (61) des kohärenten Lichts an dem vorbestimmten Detektionsort (62) in einem einzigen Pixel zu detektieren.

**9.** Vorrichtung (1) nach einem der vorangehenden Ansprüche, weiterhin umfassend eine Punktlichtquelle (521, die an dem Strahlerzeugungsort (52) angeordnet und in der Lage ist, einen divergenten einfallenden kohärenten Lichtstrahl (51) zu erzeugen.

**10.** Vorrichtung (1) nach einem der vorangehenden Ansprüche, weiterhin umfassend eine separate äussere Schicht (8), die auf der ebenen Oberfläche (21) des Substrats (2) vorgesehen ist.

**11.** Verfahren zum Erkennen von Bindungsaffinitäten, umfassend die Schritte:

- Bereitstellen einer Vorrichtung (1) nach einem der vorangehenden Ansprüche;
- Aufbringen einer Vielzahl von Zielmolekülen (32) an den Bindungsstellen (31);
- an einem vorbestimmten Strahlerzeugungsort (52) relativ zu der Vielzahl von benachbart angeordneten gekrümmten Linien (4) Erzeugen eines kohärenten Lichtstrahls (51) einer vorbestimmten Wellenlänge, der auf die Bindungsstellen (31) mit dem gebundenen Zielmolekül (32) einfällt, um an den Bindungsstellen (31) mit dem daran gebundenen Zielmolekül (32) derart gebeugt zu werden, dass gebeugte Anteile (61) des einfallenden kohärenten Lichtstrahls (51) an einem vorbestimmten Detektionsort (62) im Verhältnis zu der Vielzahl von benachbart angeordneten gekrümmten Linien (4) zu interferieren mit einer Differenz in der optischen Weglänge, die ein ganzzahliges Vielfaches der vorbestimmten Wellenlänge des kohärenten Lichts (51) ist, um ein Signal bereit zu stellen, das repräsentativ für die Bindungsstellen (31) mit dem daran gebundenen Zielmolekül (32) an dem vorbestimmten Detektionsort (62) ist;
- an dem vorbestimmten Detektionsort (62) Messen des Signals, das für die Bindungsstellen (31) mit dem daran gebundenen Zielmolekül (32) repräsentativ ist; und
- Detektieren der Bindungsaffinität der Bindungsstellen (31) und des Zielmoleküls (32) durch Vergleichen des für die Bindungsstellen (31) mit dem daran gebundenen Zielmolekül (32) repräsentativen Signals mit einem Referenzsignal, das nur für die Bindungsstellen (31) repräsentativ ist.

**12.** Verfahren nach Anspruch 11, wobei das Verfahren vor dem Aufbringen der Vielzahl von Zielmolekülen (32) an die Bindungsstellen (31) die Schritte umfasst:

- an dem vorbestimmten Strahlerzeugungsort (52) Erzeugen des einfallenden kohärenten Lichtstrahls (51), der nur an den Bindungsstellen (31) gebeugt wird derart, dass gebeugte Anteile (61) des einfallenden kohärenten Lichtstrahls (51) an dem vorbestimmten Detektionsort (62) interferieren mit einer Differenz in der optischen Weglänge, die ein ganzzahliges Vielfaches der vorbestimmten Wellenlänge des kohärenten Lichts (51) ist, um ein nur für die Bindungsstellen (31) repräsentatives Signal an dem vorbestimmten Detektionsort (62) bereit zu stellen; und
- an dem vorbestimmten Detektionsort (62) Messen des nur für die Bindungsstellen (31) repräsentativen Signals, um das Referenzsignal bereit zu stellen.

**13.** Verfahren nach einem der Ansprüche 11 oder 12, weiterhin umfassend den Schritt:

- Aufbringen einer Vielzahl komplementärer Bindungsmoleküle (311) auf die ebene Oberfläche (21) des Substrats, wobei die komplementären Bindungsmoleküle (311) in der Lage sind, an die Zielmoleküle (32) anzubinden, die an die Bindungsstellen (31) gebundenen sind, wobei die komplementären Bindungsmoleküle (311) einen Streuungsverstärker (312) umfassen.

## Revendications

**1.** Dispositif (1) destiné à l'utilisation dans la détection d'affinités de liaison, le dispositif (1) comprenant un substrat (2) dépourvu d'un guide d'onde, le substrat (2) comprenant une surface plane (21) comportant disposée sur celle-ci une pluralité de sites de liaison (31) pouvant se lier à une molécule cible (32), le dispositif comprenant encore un emplacement de génération de faisceau (52) préétabli, un emplacement de détection (62) préétabli et un moyen d'arrêt de faisceau (7) qui est disposé sur la surface du substrat, pour, en fonctionnement, empêcher la propagation des parties non diffractées (63) d'un faisceau de lumière cohérente (51) d'une longueur d'onde préétablie engendré à l'emplacement de génération de faisceau (52) et incident en venant frapper les sites de liaison (31) avec la molécule cible liée (32), vers l'emplacement de détection (62) préétabli, **caractérisé en ce que** les sites de liaison (31) sont disposés sur la surface plane (21) le long d'une pluralité de lignes courbes (4) disposées de façon adjacente, qui sont espacées les unes des autres d'une distance pour faire en sorte que, en fonctionnement, le faisceau de lumière cohérente (51) de la longueur d'onde préétablie engendré à l'emplacement de génération de faisceau (52) préétabli, par rapport à la pluralité de lignes courbes (4) disposées de façon adjacente et incident en venant frapper les sites de liaison (31) avec la molécule cible liée (32), soit diffracté au niveau des sites de liaison (31) avec la molécule cible liée (32), d'une manière telle que les parties diffractées (61) du faisceau incident de lumière cohérente (51) interfèrent au niveau de l'emplacement de détection (62) préétabli, par rapport à la pluralité de lignes courbes (4) disposées de façon adjacente, avec une différence de longueur de trajet optique qui est un nombre entier multiple de la longueur d'onde préétablie de la lumière cohérente pour produire un signal représentatif de l'affinité de liaison des sites de liaison (31) et de la molécule cible (32),
**en ce que** le dispositif comprend encore un support (22) comportant l'emplacement de génération de faisceau (52) préétabli et l'emplacement de détection (62) préétabli disposés sur celui-ci, le support (22) étant disposé par rapport au substrat (2) de telle sorte que l'emplacement de génération de faisceau (52) préétabli et l'emplacement de détection préétabli sont disposés dans un plan commun qui est parallèle à la surface plane (21) du substrat (2),
**et en ce que** chaque ligne de la pluralité de lignes courbes (4) est disposée sur la surface plane (21) du substrat (2) de telle sorte que la longueur totale du trajet de la lumière cohérente incidente (51), de l'emplacement de génération de faisceau (52) préétabli aux sites de liaison (31) disposés sur la même ligne courbe (4) respective plus la partie diffractée (61) de la lumière cohérente, des sites de liaisons sur la même ligne courbe (4) à l'emplacement de détection préétabli, est constante pour tous les sites de liaison sur la même ligne courbe (4), les lignes courbes étant définies géométriquement dans un plan x,y au niveau de la surface plane du substrat par l'équation suivante :

$$y_j{}^2 = \left( \frac{(j_0 + j)\lambda}{2n_s} \right)^2 + \left( \frac{2\,n_s\ \xi\ x_j}{(j_0 + j)\lambda} \right)^2 - \left( f^2 + \xi^2 + x_j{}^2 \right)$$

dans laquelle

$j_0$, j représentent des nombres entiers,

$\lambda$ est la longueur d'onde dans le vide préétablie de la lumière cohérente,

$n_s$ est l'indice de réfraction du substrat,

$\xi$ est la mi-distance entre l'emplacement de génération de faisceau et l'emplacement de détection et est différent de zéro, et

f est un nombre qui correspond à la distance entre le centre des lignes courbes et l'emplacement de détection.

2. Dispositif (1) selon la revendication 1, dans lequel le substrat (2) comprend un matériau qui est transparent à la lumière cohérente de la longueur d'onde préétablie, de manière à permettre la propagation du faisceau incident de lumière cohérente (51) et des parties diffractées (61) de lumière cohérente à travers le substrat (2).

3. Dispositif (1) selon la revendication 2, dans lequel le moyen d'arrêt de faisceau (7) comprend une section non transparente (71) qui et disposée sur ou dans le substrat (2) d'une façon permettant d'empêcher la propagation des parties non diffractées (63) du faisceau incident de lumière cohérente (51) vers l'emplacement de détection (62) préétabli.

4. Dispositif (1) selon la revendication 2, dans lequel le moyen d'arrêt de faisceau (7) comprend une section anti-réfléchissante disposée au niveau de la surface plane (21) du substrat (2).

5. Dispositif (1) selon la revendication 2, dans lequel le moyen d'arrêt de faisceau (7) comprend un corps déflecteur (72) disposé sur ou dans le substrat (2), le corps déflecteur (72) ayant une surface externe courbe (721) qui est capable en fonctionnement de disperser la lumière cohérente incidente venant frapper le corps déflecteur (72) de manière à empêcher la propagation de la partie non diffractée (63) du faisceau incident de lumière cohérente vers l'emplacement de détection (62) préétabli.

6. Dispositif (1) selon l'une quelconque des revendications précédentes, dans lequel l'emplacement de génération de faisceau (52) préétabli et l'emplacement de détection (62) préétabli sont disposés sur une surface externe (23) du substrat (2) opposée à la surface plane (21), dans lequel l'emplacement de génération de faisceau (52) préétabli et l'emplacement de détection (62) préétabli sont disposés sur la surface externe (23) opposée à la surface plane (21) du substrat (2) dans un plan commun qui est parallèle à la surface plane (21) du substrat (2).

7. Dispositif (1) selon la revendication 1, dans lequel les lignes courbes adjacentes (4) de la pluralité de lignes courbes (4) sont disposées en étant espacées les unes des autres d'une distance telle que la longueur totale du trajet optique de la lumière cohérente incidente (51), de l'emplacement de génération de faisceau (52) préétabli aux sites de liaison (31) disposés sur différentes lignes courbes (4), et des parties diffractées (61) de la lumière cohérente, des sites de liaison disposés sur ces différentes lignes courbes (4) à l'emplacement de détection (62) préétabli, a une différence qui est un nombre entier multiple de la longueur d'onde préétablie de la lumière cohérente.

8. Dispositif (1) selon l'une quelconque des revendications précédentes, dans lequel l'emplacement de détection (62) préétabli est une surface (662) détectrice d'un détecteur CCD ou CMOS (621) ayant une pluralité de pixels disposés sur la surface détectrice (622) en une disposition en forme de grille, chaque pixel ayant une taille telle qu'elle lui permet de détecter moins d'une moitié de l'intensité totale distribuée spatialement des parties diffractées (61) interférentes de la lumière cohérente à l'emplacement de détection (62) préétabli dans un seul pixel.

9. Dispositif (1) selon l'une quelconque des revendications précédentes, comprenant encore une source de lumière ponctuelle (521) disposée à l'emplacement de génération de faisceau (52) et capable d'engendrer un faisceau incident divergent de lumière cohérente (51).

10. Dispositif (1) selon l'une quelconque des revendications précédentes, comprenant encore une couche externe séparée (8) fournie sur la surface plane (21) du substrat (2).

**11.** Procédé pour la détection d'affinités de liaison, comprenant les étapes consistant à :

- fournir un dispositif (1) selon l'une quelconque des revendications précédentes ;
- appliquer une pluralité de molécules cibles (32) sur les sites de liaison (31) :
- à un emplacement de génération de faisceau (52) préétabli, par rapport à la pluralité de lignes courbes (4) disposées de façon adjacente, engendrer un faisceau de lumière cohérente (51) d'une longueur d'onde préétablie et incident en venant frapper les sites de liaison (31) avec la molécule cible (32) liée, de manière à être diffracté au niveau des sites de liaison (31) auxquels est liée la molécule cible (32) de telle sorte que les parties diffractées (61) du faisceau incident de lumière cohérente (51) interfèrent en un emplacement de détection (62) préétabli, par rapport à la pluralité de lignes courbes (4) disposées de façon adjacente, avec une différence de longueur de trajet optique qui est un nombre entier multiple de la longueur d'onde préétablie de la lumière cohérente (51) pour produire un signal représentatif des sites de liaison (31) auxquels est liée la molécule cible (32) à l'emplacement de détection (62) préétabli ;
- à l'emplacement de détection (62) préétabli, mesurer le signal représentatif des sites de liaison (31) auxquels est liée la molécule cible (32) ; et
- détecter l'affinité de liaison des sites de liaison (31) et de la molécule cible (32) en comparant le signal représentatif des sites de liaison (31) auxquels est liée la molécule cible (32) à un signal de référence représentatif des sites de liaison (31) seuls.

**12.** Procédé selon la revendication 11, le procédé avant l'étape consistant à appliquer la pluralité de molécules cibles (32) sur les sites de liaison (31) comprenant les étapes consistant à :

- à l'emplacement de génération de faisceau (52) préétabli, engendrer le faisceau incident de lumière cohérente (51) qui est destiné à être diffracté au niveau des sites de liaison (31) seuls de sorte que les parties diffractées (61) du faisceau incident de lumière cohérente (51) interfèrent à l'emplacement de détection (62) préétabli, avec une différence de longueur de trajet optique qui est un nombre entier multiple de la longueur d'onde préétablie de la lumière cohérente (51) pour produire un signal représentatif des sites de liaison (31) seuls à l'emplacement de détection (62) préétabli ; et
- à l'emplacement de détection (62) préétabli, mesurer le signal représentatif des sites de liaison (31) seuls pour obtenir le signal de référence.

**13.** Procédé selon l'une quelconque des revendications 11 et 12, comprenant encore l'étape consistant à :

- appliquer une pluralité de molécules se liant complémentaires (311) sur la surface plane (21) du substrat, les molécules se liant complémentaires (311) étant capables de se lier aux molécules cibles (32) liées aux sites de liaison (31), où les molécules se liant complémentaires (311) comprennent un activateur de dispersion (312).

Fig. 1

Fig. 2

Fig. 3

Fig. 4

Fig. 5

Fig. 6

Fig. 7a

Fig. 7b

Fig. 8

Fig. 9

Fig. 10

Fig. 11

Fig. 12

Fig. 13

**EP 3 022 547 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2618130 A1
- US 2002025534 A1